(19) Europäisches Patentamt
European Patent Office
Office européen des brevets

(11) Veröffentlichungsnummer: **0 071 863**
**B1**

# (12) EUROPÄISCHE PATENTSCHRIFT

(45) Veröffentlichungstag der Patentschrift: **11.06.86**

(51) Int. Cl.⁴: **C 07 D 209/76**, A 01 N 43/38

(21) Anmeldenummer: **82106730.3**

(22) Anmeldetag: **26.07.82**

(54) **Verwendung von N-(Dichlorfluormethylthio)-3,6-endomethylen-Delta4-tetrahydrophthalimid.**

(30) Priorität: **07.08.81 DE 3131250**

(43) Veröffentlichungstag der Anmeldung: **16.02.83 Patentblatt 83/7**

(45) Bekanntmachung des Hinweises auf die Patenterteilung: **11.06.86 Patentblatt 86/24**

(84) Benannte Vertragsstaaten: **BE CH DE FR GB IT LI NL SE**

(56) Entgegenhaltungen:
**DE - B - 1 193 498**
**US - A - 2 553 770**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., V.Bodelschwingh-Strasse 42, D-5060 Bergisch-Gladbach 2 (DE)**
Erfinder: **Klauke, Erich, Dr., Eichendorffweg 8, D-5068 Odenthal (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**

EP 0 071 863 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft die Verwendung von N-(Dichlorfluormethylthio)-3,6-endomethylen-$\Delta^4$-tetrahydrophthalimid.

Die Verwendung von N-(Trihalogenmethylthio)-Verbindungen zum Schutz technischer Materialien gegen mikrobiellen Abbau ist bekannt (US 2 553 770, Journ. Agr. Food Chem. 14, 365 (1966), Fette, Seifen, Anstrichmittel 68, 272 (1966)). Sie befriedigen jedoch nicht immer, da sie besonders in einigen Anstrich- und Imprägniermitteln schlecht löslich sind.

Aus der DE-AS 11 93 498 ist weiterhin ein allgemeines Verfahren zur Herstellung von Sulfensäurederivaten, u.a. auch zur Herstellung von N-(Dichlorfluormethylthio)-3,6-endomethylen-$\Delta^4$-tetrahydrophthalimid bekannt. Außerdem ist bekannt, daß die danach hergestellten Sulfensäurederivate sich als Pflanzenschutzmittel, vor allem als Fungizide, eignen.

Gegenstand der vorliegenden Erfindung ist somit die Verwendung von N-(Dichlorfluormethylthio)-3 6-endomethylen $\Delta^4$-tetrahydrophthalimid in Anstrich- und Imprägniermitteln für Holz.

Das N-(Dichlorfluormethylthio)-3,6-endomethylen-$\Delta^4$ -tetrahydrophthalimid der Formel (I)

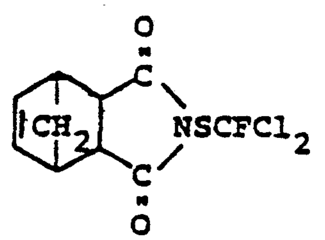

(I)

kann hergestellt werden, indem man 3,6-Endomethylen-$\Delta^4$-tetrahydrophthalimid der Formel (II)

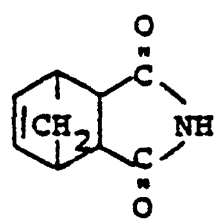

(II)

mit Dichlorfluormethansulfenylchlorid der Formel

$Cl_2F$ C S Cl

in Gegenwart eines säurebindenden Mittels in Lösung umsetzt. 090003 Das als Ausgangsmaterial zu verwendene 3,6-Endomethylen-$\Delta^4$-tetrahydrophthalimid ist bekannt und läßt sich in einfacher Weise aus dem durch Diensynthese von Cyclopentadien mit Maleinsäureanhydrid zugänglichen 3,6-Endomethylen- $\Delta^4$ -tetrahydrophthalsäureanhydrid und Ammoniak herstellen. Je nach Temperaturführung liegt die Verbindung in der Exo- oder Endo-Form vor (H. Wollweber, Diels-Alder-Reaktion, Georg Thieme Verlag Stuttgart, 1972, Seite 13).

Säurebindende Mittel für das dises Verfahren können beispielsweise Natriumhydroxid, Natriumcarbonat, Triethylamin oder Pyridin sein.

Lösungsmittel für dieses Verfahren können beispielsweise Kohlenwasserstoffe, wie z.B. Toluol, Chlorkohlenwasserstoffe, wie z.B. Chlorbenzol, Ether wie z.B. Dioxan, oder Wasser sein.

Das Verfahren zur Herstellung von N-(Dichlorfluormethyl-thio)-3,6-endomethylen-$\Delta^4$-tetrahydrophthalimid wird im allgemeinen im Temperaturbereich von 0 bis 100° C, bevorzugt von 20 bis 50° C, durchgeführt.

Im allgemeinen wird das Verfahren bei Normaldruck durchgeführt.

Das N-(Dichlorfluormethylthio)-3,6-endomethylen-$\Delta^4$-tetra-hydrophthalimid wird erfindungsgemäß als Wirkstoff zur Bekämpfung von Mikroorganismen, im besonderen in Anstrich- und Imprägniermitteln, für Holz verwendet werden.

Mikroorganismen, die einen Abbau oder eine Veränderung der Anstrich- und Imprägniermittel bewirken können, sind beispielsweise Bakterien, Pilze, Hefen, Algen, Schleime und Viren. Vorzugsweise wirkt-N-(Dichlorfluormethylthio)-3,6-endomethylen-$\Delta^4$-tetrahydrophthalimid gegen Schimmelpilze, holzverfärbende Pilze und holzzerstörende Pilze (Basidiomyceten).

Es seien beispielsweise Mikroorganismen der folgenden Gattungen genannt:

Alternaria, wie Alternaria tenuis; Aspergillus, wie Aspergillus niger; Chaetomium, wie Chaetomium globosum; Coniophora, wie Coniophora cerebella; Lentinus, wie Lentinus tigrinus; Penicillium, wie Penicillium glaucum; Polyporus, wie Polyporus versicolor; Pullularia, wie Pullularia pullulans; Sclerophoma, wie Sclerophoma pityophila; Trichoderma, wie Trichoderma viride; Escherichia, wie Escherichia coli; Staphylococcus, wie Staphylococcus aureus.

Je nach seinem Anwendungsgebiet kann der Wirkstoff in die üblichen Formulierungen überführt werden, wie Lösungen, Emulsionen, Suspensionen, Prüfer, Pasten und Granulate. Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Veiwendung von oberflächenaktiven Mitteln, wie Emulgatoren und/oder Dispergiermitteln, wobei beispielsweise im Falle der Benutzung von Verstreckmitteln, gegebenenfalls organische Lösungsmittel als Hilfslösungsmittel verwendet werden können. Organische Lösungsmittel für den Wirkstoff können beispielsweise Alkohole, wie niedere aliphatische Alkohole ($C_1$ bis etwa $C_6$), vorzugsweise Ethanol oder Isopropanol, Ketone wie Aceton oder Methylethylketon, flüssige Kohlenwasserstoffe, wie Benzinfraktionen, chlorierte Kohlenwasserstoffe, wie 1,2-Dichlorethan, sein.

Die Anwendungskonzentration des Wirkstoffes richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegt die Anwendungskonzentration im Bereich von 0,001 bis 5 Gew.-%, vorzugsweise von 0,1 bis 1,0 Gew.-%, bezogen auf das zu schützende Material.

N-(Dicklorflormethylthis)-3,6-endsmethylen-$\Delta^4$-Tetrahydro-phthalimid kann auch in Mischung mit anderen bekannten Wirkstoffen verwendet werden. Beispielsweise seien die folgenden Wirkstoffe genannt: Benzimidazolyl-methylcarbamat, Tetramethyl-thiuramdisulfid, Zink-dimethyl-dithiocarbamat, N-Fluordichlormethylthiophthalimid und N,N-Dimethyl-N'-phenyl-(N'fluordichlormethylthio)-sulfamid, N-Methylolamide und Phenolderivate, wie p-Chlor-m-kresol, 2-Phenyl-phenol und (2,2'-Dihydroxy-5,5'-dichlor)-diphenylmethan.

## Herstellungsbeispiel

### Beispiel 1

82 g (0,5 Mol) 3 6-Endomethylen- $\Delta^4$-tetrahydrophthalimid (Schmelzpunkt 184-186°C) mit 85 g (0,5 Mol) Dichlorfluormethansulfenylchlorid werden in 400 ml Cyclohexan vorgelegt. Hierzu tropft man 56 g (0,55 Mol) Triethylamin und läßt die Temperatur bis etwa 50°C ansteigen. Man rührt kurze Zeit nach und saugt das Reaktionsprodukt und das angefallene Triethylaminhydrochlorid bei Raumtemperatur ab. Man wäscht das Kristallisat mit Wasser und erhält nach dem Trocknen bei 50 bis 60°C 112 g = 75 % der Theorie des Reaktionsproduktes. Schmelzpunkt 102-104°C.

## Anwendungsbeispiele

### Beispiel 2

Zum Nachweis der Wirksamkeit gegen Pilze werden die minimalen Hemm-Konzentrationen (MHK) der gemäß Beispiel 1 erhältliden Substanz bestimmt:

Ein Agar, der aus Bierwürze und Pepton hergestellt wird, wird mit der nach Beispiel 1 erhältliden Substanz in Konzentrationen von 0,5 mg/l bis 5000 mg/l versetzt.

Nach Erstarren des Agars erfolgt Kontamination mit Reinkulturen der in der Tabelle aufgeführten Testorganismen. Nach 2-wöchiger Lagerung bei 28°C und 60 bis 70 % relativer Luftfeuchtigkeit wird die MHK bestimmt. MHK ist die niedrigste Konzentration an Wirkstoff bei der keinerlei Bewuchs durch die verwendete Mikrobenart erfolgt, sie ist in der nachstehenden Tabelle angegeben.

Tabelle I: Angabe der MHK-Werte in mg/l bei der Einwirkung der unten angegebenen Wirkstoffe auf Pilze.
Wirkstoff A: N-(Dichlorfluormethylthio)-3,6-endomethy-len-tetrahydrophthalimid;
Wirkstoff B: N,N-Dimethyl-N'-phenyl-N'-(dichlorfluor
(Vergleich) methylthio)-sulfamid;

| Testpilze | Wirkstoffe A | B |
|---|---|---|
| Alternaria tenuis | 10 | 20 |
| Aspergillus niger | 200 | 50 |
| Cladosporium herbarium | 7 | 35 |
| Coniophora cerebella | < 1 | 10 |
| Paecilomyces varioti | 10 | 20 |
| Penicillium citrinum | 7 | 150 |
| Penicillium glaucum | 10 | 35 |
| Pullularia pullulans | 5 | 20 |
| Stachybotris atra C. | 15 | 50 |
| Trichoderma viride | 200 | 5 000 |

**Beispiel 3**

(Wirkung gegen Bakterien)

Ein Agar, der als Nährmedium Bouillon enthält, wird mit den in Tabelle II angegebenen Wirkstoffen A und B (Beispiel 2) in Konzentrationen von 1 bis 5000 ppm versetzt. Darauf infiziert man das Nährmedium jeweils mit Escherichia coli oder Staphylococcus aureus und hält das infizierte Medium 2 Wochen bei 28°C und 60 bis 70 % relativer Luftfeuchtigkeit. Die MHK ist die niedrigste Konzentration an Wirkstoff, bei der keinerlei Bewuchs durch die verwendete Mikrobenart erfolgt.

Die MHK-Werte sind in Tabelle II wiedergegeben.

**Tabelle II**

Angabe der MHK-Werte in mg/l bei der Einwirkung der unten angegebenen Wirkstoffe auf Bakterien.

| | Wirkstoffe MHK (mg/l) A | MHK (mg/l) B |
|---|---|---|
| Escherichia coli | 200 | 500 |
| Staphylococcus aureus | 100 | 5 000 |

**Beispiel 4**

Aus der folgenden Tabelle geht hervor, daß N-(Dichlorfluormethylthio)-3,6-endomethy-len-$\Delta^4$-tetrahydrophthalimid (Substanz A) über sehr gute Löslichkeitseigenschaften verfügt, vor allem im Vergleich zu dem handelsüblichen Produkt N,N-Dimethyl-N'-phenyl-N'-(dichlorfluormethylthio)-sulfamid (Substanz B). Diese überraschend guten Löslichkeitseigenschaften im Verbund mit der überlegenen Wirksamkeit von Substanz A erleichtern die Anwendung in Anstrich- und Imprägniermitteln in technisch fortschrittlicher Weise.

Löslichkeit von Substanz A im Vergleich zu Substanz B (g/l bei 20°C):

| Lösungsmittel | A | B |
|---|---|---|
| Ethylacetat | 330 | 117 |
| Solvesso 100 | 200 | 75 |
| Shellsol AB | 140 | 60 |
| Xylol | 250 | 65 |

**Patentansprüche:**

1) Verwendung von N-(Dichlorfluormethylthio)-3,6-endo-methylen-$\Delta^4$-tetrahydrophthalimid in Anstrich- und Imprägniermitteln für Holz.

2) Verwendung nach Anspruch 1, dadurch gekennzeichnet, daß 0,001 bis 5 Gew.-% des Wirkstoffs, bezogen auf das zu schützende Material, eingesetzt wird.

**Claims**

1. Use of N-(dichloroflouromethylthio)-3,6-endomethylene-$\Delta$ $^4$-tetrahydrophthalimide in coating and impregnating agents for wood.

2. Use according to Claim 1, characterised in that 0.001 to 5% by weight of the active compound, based on the material to be protected, is used.

**Revendications**

1. Utilisation de la N-(dichlorofluorométhyl-thio)-3,6-endométhylène-$\Delta^4$-tétrahydrophtalimide dans des agents de peinture et d'imprégnation pour le bois.

2. Utilisation selon la revendication 1, caractérisée en ce qu'on utilise 0,001 à 5% en poids de la matière active par rapport au matériau à protéger.